# EUROPÄISCHE PATENTANMELDUNG

(11) **EP 0 822 258 A2**
(43) Veröffentlichungstag der Anmeldung: **04.02.1998**
(21) Anmeldenummer: 97112965.5
(22) Anmeldetag: 28.07.1997
(51) Int. Cl.: C12N 15/70

(54) **Positiver Selektionsvektor auf Basis des caps-Gens, pCAPs-Vektor und seine Verwendung**

(30) Priorität: 29.07.1996 DE 19630617
(71) Anmelder: Boehringer Mannheim GmbH, 68305 Mannheim-Waldhof (DE)
(72) Erfinder: Schlieper, Daniel, 50676 Köln (DE); Sobek, Harald, Dr., 82377 Penzberg (DE); Schmidt, Manfred, 82377 Penzberg (DE); von Wilcken-Bergmann, Brigitte, Dr. rer. nat., 50733 Köln (DE); Müller-Hill, Benno, Prof. Dr., 50674 Köln (DE)
(74) Vertreter: Böhm, Brigitte, Dipl.-Chem. Dr.

(57) **Zusammenfassung**

Ein erfindungsgemäßer Vektor mit positiver Selektionsmöglichkeit zur Klonierung einer DNA-Sequenz in einer mit dem Vektor transformierten Wirtszelle, wobei der Vektor geeignete Restriktionsschnittstellen innerhalb eines Selektionsgens enthält, dessen funktionelle Expression durch Einfügung des Fremdgens unterbunden wird, ist dadurch gekennzeichnet, daß er als Selektionsgen ein modifiziertes *cap*-Gen enthält, welches für ein CAP-Protein mit veränderter DNA-Bindungsspezifität kodiert, dessen Expression für Adenylat-Cyclase-positive Wirtszellen letal ist.

## Beschreibung

Die vorliegende Erfindung betrifft einen Vektor mit positiver Selektionsmöglichkeit zur Klonierung einer DNA-Sequenz in einer mit dem Vektor transformierten Wirtszelle, wobei der Vektor geeignete Restriktionsschnittstellen innerhalb eines Selektionsgens enthält, dessen Expression durch Einfügung des Fremdgens unterbunden wird, Verfahren zur Konstruktion eines erfindungsgemäßen Vektors sowie die Verwendung des Vektors als positiven Selektionsvektor für die Klonierung von DNA-Sequenzen und schließlich Verfahren zur Klonierung von DNA-Sequenzen durch Insertion der DNA-Sequenz in ein Selektionsgen eines positiven Selektionsvektors und nachfolgende Replikation des Vektors in geeigneten Wirtszellen und einem Kit zur Klonierung von DNA-Sequenzen.

Für einige molekularbiologische Anwendungen ist ein isoliertes DNA-Fragment ausreichend. Bei Anwendungen wie z. B. Transkription, Mutation, Sequenzierung oder Expression ist es jedoch wichtig, einen Bakterienklon zu erhalten, der das betreffende DNA-Fragment enthält.

Bei der Klonierung von DNA werden typischerweise die zu klonierenden DNA-Fragmente durch Spaltung der DNA mit einem geeigneten Restriktionsenzym erzeugt. Die DNA-Fragmente werden anschließend mit einem Vekor, der auf geeignete Weise hergestellt wurde, ligiert und in kompetente Zellen transformiert.

Fur die Fälle, daß es sich bei der DNA um durch PCR amplifizierte DNAs handelt, können verschiedene Klonierungsstrategien angewandt werden. So können z.B. an die Enden der betreffenden Primersequenzen die Erkennungssequenzen für eine Restriktionsendonuklease eingebaut werden. Das durch PCR erzeugte DNA-Fragment trägt somit am Ende die entsprechende Restriktionssequenz und kann nach der PCR mit dem jeweiligen Restriktionsenzym gespalten werden (Kaufmann und Evans, 1990, BioTechniques 9, 304 - 306).

Eine weitere Klonierungsstrategie besteht darin, die sog. Extendase-Aktivität von DNA-Polymerasen auszunützen. Bei der Verwendung von z.B. Taq DNA Polymerase kommt es zu einer nicht template-bedingten Verlängerung des PCR-Produktes am 3'-Ende des PCR-Produktes (Clark, 1989, Nucl. Acids Res, 20, 9677 - 9686). Dabei wird bei einem Teil der PCR-Produkte bevorzugt ein einzelnes Deoxyadenosinmonophosphat an die 3'-Enden der DNA angehängt. Taq DNA Polymerase (und andere DNA Polymerasen ohne 3'-5'-Exonuklease-Aktivität) erzeugt somit ein PCR-Produkt mit einem DNA-Ende, das für eine Ligation mit einem geeigneten Vektor (mit je einem Thymidinmonophosphat an den 3'-Enden) verwendet werden kann (Mead et al. 1991, Biotechnol. 9, 657 - 663; PCT Application WO 92/06189).

Des weiteren können PCR-Produkte über eine sog. blunt-end-Ligation in einen geeigneten Vektor ligiert werden. Dieses Verfahren wird bevorzugt angewendet, wenn das betreffende PCR-Fragment mit einer Polymerase mit 3'-5'-Exonuklease-Aktivität (d.h. ohne Extendase-Aktivität) amplifiziert worden ist (Costa et al., 1994, PCR Meth. and Appl. 3, 338 - 345; Lohff und Cease, 1991, Nucl. Acids Res. 20 (1), 144).

Zur Klonierung von DNA in E.coli stehen eine Vielzahl von verschiedenen Vektoren zur Verfügung. Eine Untergruppe dieser Klonierungsvektoren, die sog. positiven Selektionsvektoren, erlauben die direkte Selektion von Klonen, die ein DNA-Fragment als Insert in dem betreffenden Vektor tragen. Das betreffende DNA-Fragment wird dabei in ein (z.B.) letales Gen durch Klonierung inseriert. Bakterienklone, die einen Vektor tragen, dessen letales Gen nicht durch Insertion eines DNA-Fragments inaktiviert wurde, exprimieren dieses letale Gen und sind somit nicht überlebensfahig. Es kommt dadurch zu einer Selektion von Klonen, die das gewünschte DNA-Fragment tragen. Vektoren mit solchen Eigenschaften werden als Suizid-Vektoren bezeichnet. Die Verwendung von Suizid-Vektoren zur positiven Selektion stellt somit eine effiziente Strategie dar, um einen unerwünschten Background an nicht rekombinanten Klonen (und somit falsch positiven Klonen), die nicht das gewünschte DNA-lnsert tragen, zu unterdrücken.

Einige positive Selektionsvektoren beruhen auf der Inaktivierung des selektionierenden Gens durch Insertion des zu klonierenden DNA-Fragments in das selektionierende Gen. Diese Gene können dabei letale Gene sein (Henrich & Plapp, 1986, Gene 42, 345 - 349; Henrich & Schmidtberger, 1995, Gene 154, 51 - 54; Bernard et al., 1994, Gene 148, 71 - 74, PCT Application WO 94/03616). Als weitere Prinzipien sind die Aufhebung einer besonderen Sensitivität gegenüber Metaboliten (Kast, 1994, Gene 138, 109 - 114), die Selektion mittels DNA- oder RNA-abbauenden Enzymen (Yaznin et al., 1996, Gene 169, 131 - 132; Ahrenholtz et al., 1994, Appl. Environ. Microbiol. 60 (10), 3746 - 3751), sowie die Selektion über instabile, lange palindromische DNA-Sequenzen (Altenbuchner et al., 1992, Methods. Enzymol. 216, 457 - 466) beschrieben.

Bei der Verwendung von Restriktionsendonukleasen zur Spaltung von Vektor-DNA und/oder DNA-Fragment erfährt man eine Einschränkung dadurch, daß bei dieser Methode es zu einer unerwünschten Spaltung innerhalb der Sequenz der zu klonierenden DNA kommen kann. Es können somit nur die Restriktionsschnittstellen in die Primer eingebaut werden, die nicht in dem betreffenden DNA-Fragment vorhanden sind. Darüber hinaus ist die Synthese dieser zusätzlichen Primersequenz kostenaufwendig. Ferner ist die Spaltungseffizienz bei diesen, an den Enden der Primer gelegenen Restriktionsschnittstellen, geringer als bei Spaltungsstellen, die innerhalb eines DNA-Abschnitts liegen (Jung et al., 1990, Nucl. Acids Res. 18 (20), 6156). Ferner kann eine vorhergehende und anschließende Reinigung der DNA nötig sein.

Bei der Verwendung eines Vektors mit einem Überhang eines Thymidinmonophosphats (T-Vektor) ergibt sich eine Einschränkung daraus, daß DNA-Polymerasen mit einer 3'- 5'-Exonuklease-Aktivität (sog. proof reading activity) kein PCR-Produkt mit einem 3 ' -Überhang eines Deoxyadenosinmonophosphats erzeugen. Diese Polymerasen erzeugen ein stumpfes Ende am 3'-Ende des PCR-Produkts (Hu, 1993, DNA Cell Biol. 12 (8), 763 - 770). Diese PCR-Produkte lassen sich nicht mit T-Vektoren klonieren. Solche Polymerasen sind z.B. die DNA-Polymerase aus Pyrococcus woesei (Pwo Polymerase) oder aus Pyrococcus furiosus (Pfu Polymerase). Solche Polymerasen können somit nicht in Kombination mit T-Vektoren verwendet werden. Eine Einschränkung ergibt sich auch bei der Verwendung von Gemischen von Polymerasen, die je aus mindestens einer Polymerase mit und einer Polymerase ohne 3'-5'-Aktivität bestehen; in diesen Fällen ist eine geringe Klonierungseffizienz zu beobachten. Des weitern ist bekannt, daß die Taq DNA Polymerase (in Abhängigkeit von der Templatesequenz) in der Lage ist, auch andere Nukleotide als Deoxyadenosinmonophosphat am 3'-Ende des PCR-Produkts anzuhängen (Hu, 1993, DNA Cell. Biol. 12 (8), 763 - 770). Derartige DNA-Fragmente sind nicht für die Klonierung mit einem T-Vektor geeignet.

Problematisch bei der Verwendung von positiven Selektionsvektoren kann u. U. eine hohe Zahl von falsch positiven Klonen, d. h. Klone ohne Insert, sein (Henrich & Plapp, 1986; Gene 42, 345 - 349; Bernard et al., 1994, Gene, 148, 71 - 74). Dies kann durch ein Wachstum von Klonen, deren Selektionsgene mutiert sind (sog. Revertanten), verursacht werden.

Desweiteren kann es bei der Verwendung von positiven Selektionsvektoren dazu kommen, daß bei der Klonierung von kürzeren DNA-Fragmenten ein vermindert (aber trotzdem) funktionsfähiges Genprodukt entsteht, das zu einem Auftreten von Klonen mit vermindertem Wachstum führt und somit die Auswertung eines Klonierungsexperiments kompliziert.

In den Fällen, bei denen die Insertion von DNA-Fragmenten die letalen Eigenschaften des Selektionsgens überhaupt nicht negativ beeinflußt, werden auch keine Klone mit Insert erhalten; d.h. eine Klonierung der betreffenden DNA-Fragmente ist somit nicht möglich. Dies ist besonders bei kleinen DNA-Fragmenten oder/und solchen Fragmenten, deren Nukleotidsequenz "in frame" mit dem Selektionsgen sind, möglich.

Nachteilig für eine Anwendung wirkt es sich ferner aus, wenn zur Ausnützung des Selektionsmechanismus komplexe Nährstoffmedien verwendet werden müssen (Kast, 1994, Gene 138, 109-114).

Es lag der vorliegenden Erfindung daher die Aufgabe zugrunde, eine Möglichkeit zu schaffen, bei der Klonierung von DNA-Sequenzen eine positive Selektion durchzuführen und die Nachteile des Standes der Technik zu vermeiden. Diese Aufgabe wird erfindungsgemäß gelöst durch einen Vektor mit positiver Selektionsmöglichkeit zur Klonierung einer DNA-Sequenz in einer mit dem Vektor transformierten Wirtszelle, wobei der Vektor geeignete Restriktionsschnittstellen innerhalb eines Selektionsgens enthält, dessen Expression durch Einfügung des Fremdgens unterbunden wird, wobei der Vektor dadurch gekennzeichnet ist, daß er als Selektionsgen ein modifiziertes *cap*-Gen (=crp-Gen, cAMP-Rezeptorprotein-Gen) enthält, welches für ein CAP-Protein mit veränderter DNA-Bindungsspezifität und codiert, dessen Expression für Adenylat-Cyclase-positive Wirtszellen letal ist. Alle E. coli Stämme sind in der Regel Adenylat-Cyclase positiv und somit für den Einsatz des erfindungsgemäßen Vektors geeignet.Die erfindungsgemäßen Vektoren eignen sich für die Verwendung zur Klonierung von DNA-Fragmenten im allgemeinen und insbesondere von DNA-Fragmenten, die durch die Methode der PCR hergestellt wurden.

Die erfindungsgemäßen Vektoren zeichnen sich dadurch aus, daß sie als positives Selektionsprinzip eine durch Mutation modifizierte Form des *cap*-Gens besitzen, das für eine modifizierte Form des Catabolite Activator Protein (CAP) kodiert. Das CAP-Protein ist ein prokaryontischer Transkriptionsfaktor, der an der Regulation des Kohlehydratstoffwechsels beteiligt ist. Besonders für E.coli ist die Funktion des CAP-Proteins gut beschrieben. Für die Funktion wird dabei cAMP benötigt (Zubay et al., 1970, PNAS 66 (1), 104-110). In einer besonders bevorzugten Ausführungsform trägt der erfindungsgemäße Vektor eine Modifikation des *cap*-Gens an einer Stelle, die nach Expression zu einer Mutante führt, die an Position 181 des Proteins einen Aminosäureaustausch enthält. In einer besonders bevorzugten Ausführungsform enthält die Mutante CAPs an Position 181 Glutamin anstelle von Glutaminsäure. Durch diese Mutation wird die Bindungsspezifität an DNA relaxiert und die Möglichkeit der DNA-Bindung erweitert. Bakterienklone, die mit einem erfindungsgemäßen Vektor transformiert sind, sind nicht überlebensfähig, da die Expression des mutierten *cap*-Gens für diese Klone letal ist. Das veränderte *cap*-Gen ist nur in Adenylatcyclase-negativen Stämmen (cya⁻) klonierbar. Bei Anwesenheit von cAMP ist das modifizierte CAP-Protein durch die Bindung an bisher nicht näher bekannte Bindungssequenzen für Bakterien letal und kann daher für einen Suizidklonierungsvektor verwendet werden.

Für eine solche Anwendung können alle, durch Mutation veränderten Formen des *cap*-Gens verwendet werden, sofern sie für veränderte CAP-Proteine codieren, welche ebenfalls die gewünschten, veränderten DNA-Bindungseigenschaften und somit letale Eigenschaften für Adenylatcyclase-positive Wirtsstämme besitzen.

Vorteile des erfindungsgemäßen Vektorssystems sind insbesondere der hohe Anteil an positiven Klonen (die das erwartete DNA-Insert enthalten), die bei Klonierungsexperimenten erhalten wurden. Des weiteren werden bei der Klonierung von kurzen DNA-Fragmenten und von solchen DNA-Fragmenten, die "in frame" mit dem Selektionsgen vorliegen, die letalen Eigenschaften des mutierten *cap*-Gens aufgehoben, d.h. solche DNA-Fragmente sind bei der Verwendung des mutierten CAP-Systems klonierbar. Des weiteren sind bei der Verwendung des mutierten CAP-Systems keine besonderen experimentellen Methoden, wie z.B. Primerdesign, Induktion (Temperatur-Shifts, IPTG) oder besondere Medienzusammensetzungen (X-Gal, komplexe Näherstoffmedien) nötig. Die erfindungsgemäßen Vektoren sind für Klonierung von DNA-Fragmenten mittels Ligation über überhängende Enden ebenso wie über glatte Enden (sticky-end- und blunt end-Ligation) geeignet. Insbesondere geeignet ist hierfür der erfindungsgemäß besonder bevorzugte Vektor pCAPs.

Ein weiterer Gegenstand der vorliegenden Erfindung ist ein Verfahren zur Herstellung eines erfindungsgemäßen Vektors, bei dem man einen Vektor, enthalten das *cap*-Gen, innerhalb des cap-Gens modifiziert, so daß ein erfindungsgemäßer Vektor mit einem entsprechend modifizierten *cap*-Gen entsteht, oder es wird ein bereits modifiziertes *cap*-Gen in einen geeigneten Vektor insertiert, wobei in beiden Fällen die erhaltenen Vektoren in Adenylatcyclase-negativen Wirtszellen kloniert werden.

Bevorzugt wird das *cap*-Gen im erfindungsgemäßen Verfahren an einer Stelle modifiziert, die zu einem Aminosäureaustausch der Aminosäure 181 des CAP-Proteins führt. In einer besonders bevorzugten Ausführungsform wird dabei Glutaminsäure durch Glutamin ersetzt. Besonders bevorzugt ist es im übrigen, vor das *cap*-Gen einen starken Promotor zu inserieren.

Ein weiterer Gegenstand der vorliegenden Erfindung ist die Verwendung eines erfindungsgemäßen Vektors als positiver Selektionsvektor für die Klonierung von DNA-Sequenzen, wobei die DNA-Sequenz innerhalb des modifizierten *cap*-Gens in den Vektor inseriert wird und man zur Klonierung Adenylatcyclase-positive Wirtszellen verwendet.

Ebenso ist ein Verfahren zur Klonierung von DNA-Sequenzen durch Insertion der DNA-Sequenz in ein Selektionsgen eines positiven Selektionsvektors und nachfolgende Replikation des Vektors in geeigneten Wirtszellen ein Gegenstand der Erfindung. Man verwendet dabei als Selektionsvektor einen erfindungsgemäßen Vektor, inseriert die zu klonierende DNA-Sequenz in eine geeignete Restriktionsschnittstelle innerhalb des modifizierten *cap*-Gens und führt die Klonierung in Adenylatcyclase-positiven Wirtszellen durch. Durch die Insertion der DNA-Sequenz in das modifizierte *cap*-Gen wird die Bildung des modifizierten CAP-Proteins verhindert. Das modifizierte *cap*-Gen jedoch würde die Klonierung in Adenylatcyclase-positiven Wirtszellen verhindern, da es letale Auswirkungen auf die Wirtszellen hat und diese damit absterben würden. Durch Insertion der DNA-Sequenz wird das CAP-Protein jedoch nicht mehr in funktionsfähiger Form exprimiert, weshalb die Klonierung auch in Adenylatcyclase-positiven Wirtszellen erfolgen kann. Es wird erfindungsgemäß also vermieden, daß falsch-positive Klone entstehen, da diese falsch-positiven Klone die Wirtszelle töten würden.

Besonders bevorzugt wird das erfindungsgemäße Verfahren für DNA-Sequenzen angewandt, die durch PCR-Technik hergestellt wurden. Man erhält mit Hilfe des erfindungsgemäßen Verfahrens bevorzugt Klone, die die gewünschte DNA-Sequenz enthalten. Im übrigen wird auf die oben bereits erwähnten Vorteile des erfindungsgemäßen Vektorsystems und seiner Verwendung verwiesen.

Ein weiterer Gegenstand der vorliegenden Erfindung ist ein Reagenzienkit zur selektiven Klonierung von DNA-Sequenzen, enthaltend
a) einen erfindungsgemäßen Vektor sowie
b) Adenylatcyclase-positive Wirtszellen.
Der erfindungsgemäße Reagenzienkit ermöglicht es, eine Klonierung von DNA-Sequenzen mit Hilfe des erfindungsgemäßen Systems und Verfahrens durchzuführen. Besonders bevorzugt werden dabei durch PCR erhaltene DNA-Sequenzen kloniert.

In einer besonders bevorzugten Ausführungsform der Erfindung enthält der Reagenzienkit zusätzlich geeignete Restriktionsendonukleasen zur Insertion der DNA-Sequenz in das Selektionsgen des erfindungsgemäßen Vektors.

Die folgenden Beispiele erläutern in Verbindung mit den Abbildungen die Erfindung weiter.

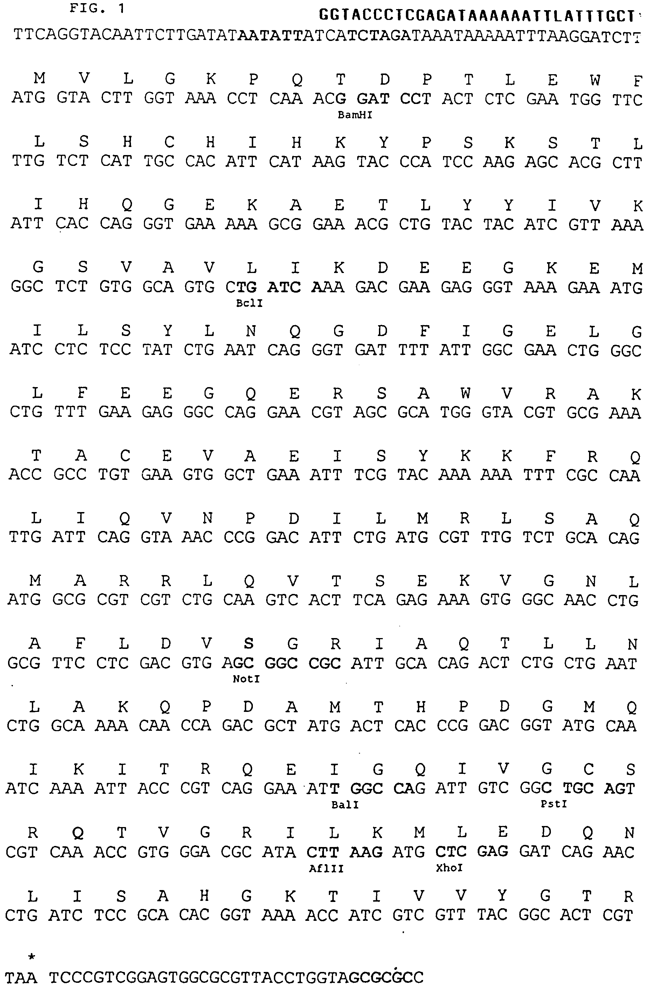

- Abb. 1: zeigt dabei die DNA- und Aminosäure-Sequenz des mutier ten *cap*-Gens,

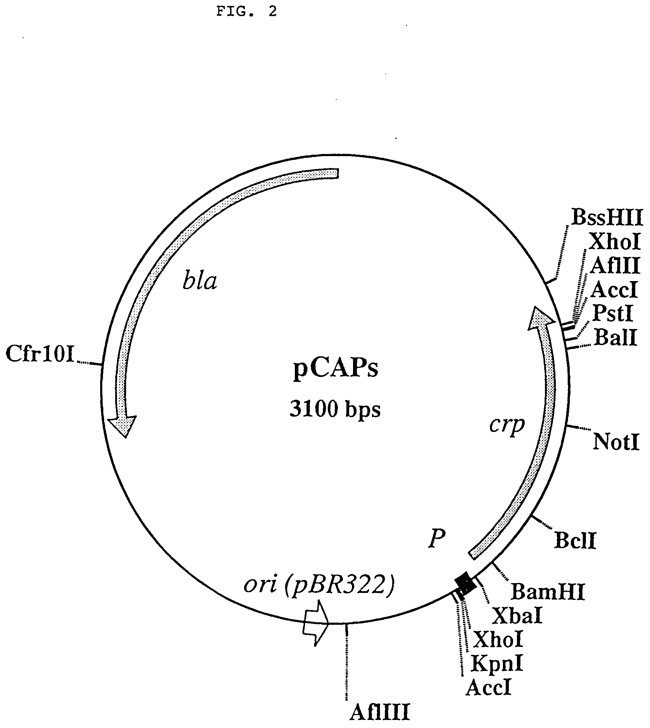

- Abb. 2: zeigt eine schematische Darstellung des Vektors pCAPs,

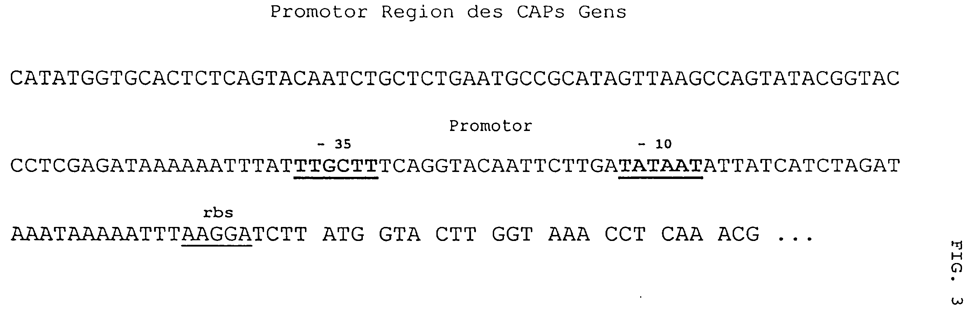

- Abb. 3: zeigt die Promotorsequenzen von pCAPs.

### Beispiel 1

### Vektorkonstruktion

Das cap-Gen wurde mit EcoRV/HindIII aus dem Vektor pHA7 gespalten (Aiba et al., 1982, Nucl. Acids Res. 10, 1345) und anschließend in den Vektor pEE4/StuI/HindIII kloniert. Aus diesem Vektor pEE4-CAP wurde das cap-Gen mittels EcoRI/HindIII gespalten und in pUC19/EcoRVHindIII kloniert. Anschließend wurde das cap-Gen hinter den synthetischen Promotor des Vektors pWB100 (Lehming et al., 1987, EMBO J. 6, 3345 - 3353) kloniert. Dazu wurde pUC l9-CAP mit HindIII linearisiert, die überstehenden 5'-Einzelstrangenden mit Klenow-Fragment der DNA-Polymerase I aufgefüllt, mit CfrI verdaut, das Fragment gereinigt und in den mit CfrI/EcoRV gespaltenen Vektor pWB100 kloniert. Dies ergibt den Vektor pBG1. Dieser Vektor wurde durch Einklonieren einer vom piWiT10 Promotor abgeleiteten stärkeren Promotorsequenz weiter modifiziert. Der erhaltene Vektor ist pCAP. Der Vektor pCAPs wurde durch Mutation des cap-Gens in der Aminosäureposition 181 (Glutaminsäure zu Glutamin) erhalten.

### Beispiel 2

### Klonierung von DNA-Fragmenten

### Allgemeine Methoden:

Die Herstellung von kompetenten E.coli-Zellen (z.B. E.coli JM83, XL1-Blue, DH5α, BMH8117) für die Transformation, sowie die Transformation der Zellen erfolgte nach Standardmethoden (Hanahan, 1983, J. Mol. Biol. 166, 557 - 580). Ebenso wurden für die Klonierung kommerziell erhältliche kompetente Zellen verwendet, z. B. (E.coli XL1-Blue MRF' kan, Stratagene). Die Zellen wurden auf LB-Medium mit Ampicillin (100-200 µg/ml) ausplattiert.

Die Spaltung von DNA (Vektor oder DNA-Fragment) mit Restriktionsendonukleasen erfolgte nach den Vorgaben der jeweiligen Hersteller.

Die Ligation von linearisiertem Vektor und DNA-Fragment erfolgte nach Standardmethoden (Maniatis et al. 1982, Molecular cloning. A laboratory manual, Cold Spring Harbour Laboratory Press, Cold Spring Harbour, NY). Ebenso wurde dafür ein kommerziell erhältlicher Kit verwendet (z. B. Rapid Ligation Kit, Boehringer Mannheim).

Die Amplifikationen von DNA mittels PCR wurden mit den entprechenden DNA Polymerasen nach den Vorgaben des Herstellers durchgeführt.

### Beispiel für blunt end-Klonierung:

Der Vektor pCAPs wird durch Verdau mit dem Restriktionsenzym BalI nach Vorgaben des Herstellers linearisiert. Dieses linearisierte Plasmid (5 ng) wird mit einem 0.25 kb langen, isolierten StuI-Fragment des Vektors piWiT1OWL1 (von Wilcken-Bergmann et al. 1986, EMBO J. 5 (12), 3219 - 3225) in 10-fach molarem Überschuß ligiert. Anschließend werden kompetente Zellen von E.coli DH5α damit transformiert, auf LB-Medium ausplattiert und über Nacht bei 37 °C inkubiert. Von den erhaltenen Klonen werden jeweils 5 ml-Kulturen angesetzt, über Nacht angezogen und davon Plasmidpräparationen hergestellt. Zur Identifizierung der Plasmide werden Restriktionsspaltungen mit den Enzymen XbaI/SphI durchgeführt. In dem hier beschriebenen Beispiel ergab die Analyse von 102 untersuchten Klonen eine Anzahl von 92 Klonen mit dem Insert.

### Beispiel fur sticky end-Klonierung:

Der Vektor pCAPs wird durch Verdau mit dem Restriktionsenzym PstI nach Vorgaben des Herstellers linearisiert. Dieses linearisierte Plasmid (5 ng) wird mit einem 0.25 kb langen, isolierten PstI-Fragment des Vektors piWiTlOWLI (von Wilcken-Bergmann et al. 1986, EMBO J. 5 (12), 3219 - 3225) in 10-fachem Überschuß ligiert. Anschließend werden kompetente Zellen von E.coli DH5α damit transformiert, auf LB-Medium ausplattiert und über Nacht bei 37 °C inkubiert. Von den erhaltenen Klonen werden jeweils 5 ml-Kulturen angesetzt, über Nacht angezogen und davon Plasmidpraparationen hergestellt. Zur Identifizierung der Plasmide werden Restriktionsspaltungen mit den Enzymen XbaI/StuI durchgeführt. In dem hier beschriebenenen Beispiel ergab die Analyse durch Agarose-Gelelektrophorese bei 108 untersuchten Klonen eine Anzahl von 100 Klonen mit dem Insert.

### Beispiel für die Klonierung von PCR-Produkten:

Die PCR-Produkte wurden durch Amplifikation eines 0.5 Kb Fragments aus Lambda-DNA hergestellt. Für die Amplifikation wurden unterschiedliche Polymerasen bzw. Mischungen davon verwendet. Es wurden dabei eine Polymerase ohne 3'-5'-Exonuklease-Aktivität (Tag DNA Polymerase), eine Polymerase mit 3'-5'-Exonuklease-Aktivität (Pwo DNA Polymerase) sowie eine Mischung dieser beiden Polymerasen (Expand High Fidelity PCR System) verwendet.

Die für die PCR verwendeten Primer haben folgende Sequenzen:
Forward primer: 5'-GAT GAG TTC GTG TCC GTA CAA CT-3
Reverse primer: 5'-GGT TAT CGA AAT CAG CCA CAG CG-3

Die PCR-Bedingungen waren:
1 ng Lambda-DNA
20 µM Primer (forward, reverse)
0.2 mM dNTPs
10 µl PCR-Puffer (geeignet für die jeweils verwendeten Polymerasen)

An Polymerasen wurden jeweils verwendet:
2.5 units Taq DNA Polymerase
2.5 units Pwo DNA Polymerase
1.75 units Expand High Fidelity PCR System
Auf 100 µl mit bidest. Wasser aufgefüllt.

Die Zyklusbedingungen waren wie folgt:
2 min bei 95 °C; 25 Zyklen mit: 1 min bei 94 °C, 1 min bei 50 °C, 3 min bei 72 °C; 4 min bei 72 °C

Die PCR-Fragmente wurden nach der Amplifikation aufgereinigt (High Pure PCR Product Purification Kit, Boehringer Mannheim).

Für die Ligation wurde 0.1 - 10 ng Vektor (linearisiert mit BalI) eingesetzt, typischerweise 1 ng. Es wurden 10 - 500 ng des gereinigten PCR-Fragments verwendet, in typischen Experimenten 100 - 400 ng. Die Ligation wurde nach dem Protokoll des Rapid Ligation Kits durchgeführrt. Für die anschließende Transformation wurde 1/10 Vol. des Ligationsansatzes verwendet (0.1 ng Vektor). 40 µl der kompetenten Zellen (E.coli XLl-Blue MRF kan) wurden nach Vorgaben des Herstellers verwendet (Gesamtvolumen 400 µl). Von den transformierten Zellen wurden auf LB-Platten (Ampicillin, 100 µg/ml) 100 µl ausplattiert und über Nacht bei 37°C inkubiert. Die erhaltenen Klone wurden in LB-Medium (Ampicillin, 150 µg/ml) über Nacht bei 37°C gezüchtet. Die Plasmid-DNA wurde nach Standardmethoden isoliert (Maniatis et al. 1982, Molecular cloning. A laboratory manual, Cold Spring Harbour Laboratory Press, Cold Spring Harbour, NY). Die isolierten Plasmide wurden durch Restriktionsspaltung mit Acc I analysiert. Das Ergebnis eines typischen Experiments ist in Tabelle 1 dargestellt:

**Tab. 1**

| pCAPs (Vektor in Ligat.) | PCR-Fragment (verwendete Pol.) | Kolonien/Platte | Pos. Klone/analys. Klone |
|---|---|---|---|
| 1 ng | 400 ng (Taq DNA Pol.) | 2 | 2/2 |
| 1 ng | 100 ng (Taq DNA Pol.) | 4 | 3/4 |
| l ng | 400 ng (Pwo DNA Pol.) | 62 | 18/18 |
| 1 ng | 100 ng (Pwo DNA Pol.) | 193 | 48/48 |
| 1 ng | 400 ng (Expand High Fid.) | 9 | 9/9 |
| 1 ng | 100 ng (Expand High Fid.) | 10 | 7/10 |

Für die blunt end-Klonierung von PCR-Fragmenten (die z. B. mittels Pwo DNA Polymerase erzeugt wurden) ist der Vektor pCAPs aufgrund der hohen Anzahl an erhaltenen Klonen sowie dem hohen Anteil an positiven Klonen (in dem aufgeführten Beispiel 100 %) besonders geeignet.

Bei der Klonierung von PCR-Fragmenten, die mittels einer Mischung von Polymerasen (Expand High Fidelity PCR System) hergestellt werden, wird ein hoher Anteil von positiven Klonen erhalten, wenn in der Ligationsreaktion ein hohes Insert/Vektor-Verhältnis vorliegt.

Bei der Klonierung von PCR-Fragmenten, die mittels Taq DNA Polymerase hergestellt wurden, ist es auch möglich, eine, wenn auch geringere, Anzahl von Klonen zu erhalten. In diesem Fall werden solche PCR-Produkte der Taq DNA Polymerase ligiert, die keinen A-Überhang besitzen.

In diesem Zusammenhang ist bekannt, daß durch ein anschließendes sog. polishing der PCR-Produkte (ein Abbau des A-Überhangs durch die 3' - 5'-Exonuklease-Aktivität von Polymerasen) die Effizienz einer Klonierung von PCR-Fragmenten, die mittels Taq DNA Polymerase hergestellt worden sind, verbessert werden kann (Costa & Weiner, 1994, Nucl. Acids Res. 22 (12), 2423).

### Beispiel 3

### Beispiel für die Klonierung von ungereinigten PCR-Produkten

Neben der Klonierung von gereinigten PCR-Produkten wurden auch ungereinigte PCR-Produkte kloniert, d.h. es wurde ein Aliquot des PCR-Ansatzes direkt in die Ligationsreaktion eingesetzt. Es wurden dazu blunt end PCR-Produkte durch Amplifikation eines 0,5 kb Fragments aus Lambda-DNA mit Pwo DNA Polymerase hergestellt.

Die für die CPR verwendeten Primer haben folgende Sequenzen:
Forward primer: 5'-GAT GAG TTC GTG TCC GTA CAA CT-3'
Reverse primer: 5'-GGT TAT CGA AAT CAG CCA CAG CG-3'

Die PCR-Bedingungen waren:
1 ng Lambda-DNA
20 µM Primer (forward, reverse)
0,2 mM dNTPs
10 µl PCR-Puffer (geeignet für Pwo DNA Polymerase)
2,5 units Pwo DNA Polymerase

Auf 100 µl mit bidest. Wasser aufgefüllt.

Die Zyklusbedingungen waren wie folgt:
2 min bei 95°C; 25 Zyklen mit: 1 min bei 94°C, 1 min bei 50°C, 3 min bei 72°C, 4 min bei 72°C.

Die anschließende Ligation wurde nach dem Protokoll des Rapid Ligation Kits (Boehringer Mannheim , Kat.Nr. 1635-379) durchgeführt. Für die Ligation wurde 0,1 - 10 ng Vektor (linearisiert Mlu NI) eingesetzt, typischerweise 1 ng. Die PCR-Fragmente wurden nach der Amplifikation ungereinigt für die Ligation verwendet. Es wurden 1 - 5 µl des PCR-Ansatzes in der Ligation verwendet (typischerweise 1 µl) Für die anschließende Transformation wurde 1/10 Vol. des Ligationsansatzes verwendet (0,1 ng Vektor). 40 µl der kompetenten Zellen (E.coli XL 1-Blue MRF' kan, Stratagene) wurden nach Vorgaben des Herstellers verwendet (Gesamtvolumen 400 µl). Von den transformierten Zellen wurden auf LB-Platten (Ampicillin, 100 µg/ml) 100 µl ausplattiert und über Nacht bei 37°C inkubiert. Die erhaltenen Klone wurden in LB-Medium (Ampicillin, 150 µg/ml) über Nacht bei 37°C gezüchtet. Die Plasmid-DNA wurde nach Standardmethoden isoliert (Maniatis et al., 1982, Molecular cloning. A laboratory manual, Cold Spring Harbour Laboratory Press, Cold Spring Harbour, NY). Die isolierten Plasmide wurden durch Restriktionsspaltung mit Acc I und anschließender Agarosegelelektrophorese analysiert. Dabei zeigten > 95 % der analysierten Klone (92 von 96) das 0,5 kb PCR Fragment als Insert.

## Patentansprüche

1. Vektor mit positiver Selektionsmöglichkeit zur Klonierung einer DNA-Sequenz in einer mit dem Vektor transformierten Wirtszelle, wobei der Vektor geeignete Restriktionsschnittstellen innerhalb eines Selektionsgens enthält, dessen funktionelle Expression durch Einfügung des Fremdgens unterbunden wird,
**dadurch gekennzeichent,**
daß er als Selektionsgen ein modifiziertes *cap*-Gen enthält, welches für ein CAP-Protein mit veränderter DNA-Bindungspezifität kodiert, dessen Expression für Adenylat-Cyclase-positive Wirtszellen letal ist.

2. Vektor nach Anspruch 1,
**dadurch gekennzeichnet,**
daß die Modifikation des *cap*-Gens zu einem Aminosäureaustauch von Aminosäure 181 des CAP-Proteins führt.

3. Vektor nach Anspruch 2,
**dadurch gekennzeichnet,**
daß das CAP-Protein als Aminosäure 181 Glutamin enthält.

4. Vektor nach einem der Ansprüche 1 bis 3,
**dadurch gekennzeichnet,**
daß er in Transkriptionsrichtung vor dem Selektionsgen einen Promotor enthält.

5. Vektor pCAPs

6. Verfahren zur Herstellung eines Vektors gemäß einem der Ansprüche 1 bis 5,
**dadurch gekennzeichnet,**
daß man einen Vektor enthaltend das *cap*-Gen innerhalb des *cap*-Gens modifiziert, oder ein bereits modifiziertes cap-Gen in einen geeigneten Vektor insertiert, und den Vektor in Adenylat-Cyclase-negativen Wirtszellen repliziert bzw. exprimiert.

7. Verfahren nach Anspruch 6,
**dadurch gekennzeichnet,**
daß man das *cap*-Gen an einer Stelle modifiziert, die zu einem Aminosäureaustausch der Aminosäure 181 des CAP-Proteins führt.

8. Verfahren nach Anspruch 7,
**dadurch gekennzeichnet,**
daß man einen Austausch von Glutaminsäure durch Glutamin bewirkt.

9. Verfahren nach einem der Ansprüche 6 bis 8,
**dadurch gekennzeichnet,**
daß man vor das *cap*-Gen einen starken Promotor inseriert.

10. Verwendung eines Vektors gemäß einem der Ansprüche 1 bis 5 als positiven Selektionsvektor für die Klonierung von DNA-Sequenzen,
**dadurch gekennzeichnet,**
daß die DNA-Sequenz innerhalb des modifizierten *cap*-Gens in den Vektor insertiert wird und man zur Klonierung Adenylat-Cyclase-positive Wirtszellen verwendet.

11. Verfahren zur Klonierung von DNA-Sequenzen durch Insertion der DNA-Sequenz in ein Selektionsgen eines positiven Selektionsvektors und nachfolgende Replikation des Vektors in geeigneten Wirtszellen,
**dadurch gekennzeichnet,**
daß man als Selektionsvektor einen Vektor gemäß einem der Ansprüche 1 bis 5 verwendet, die DNA-Sequenz in eine geeignete Restriktionsschnittstelle innerhalb des modifizierten *cap*-Gens insertiert und die Klonierung in Adenylat-Cyclase-positiven Wirtszellen durchführt.

12. Verfahren nach Anspruch 12,
**dadurch gekennzeichnet,**
daß die DNA-Sequenz durch PCR hergestellt wurde.

13. Kit zur selektiven Klonierung von DNA-Sequenzen, insbesondere von durch PCR erhaltener DNA-Sequenzen, enthaltend:
a) einen Vektor gemäß einem der Ansprüche 1 bis 5
b) Adenylat-Cyclase-positive Wirtszellen.

14. Kit nach Anspruch 13,
**dadurch gekennzeichnet,**
daß er zusätzlich geeignete Restriktionsendonukleasen zur Insertion der DNA-Sequenz in das Selektionsgen des Vektors enthält.
